# EUROPEAN PATENT APPLICATION

(11) **EP 1 731 550 A1**
(43) Date of publication of application: **13.12.2006**
(21) Application number: 05727469.8
(22) Date of filing: 31.03.2005
(51) Int. Cl.: C08G 85/00, A61K 33/00, B82B 1/00, C01B 31/02

(54) **NOVEL WATER-SOLUBLE FULLERENE, PROCESS FOR PRODUCING THE SAME AND ACTIVE OXYGEN GENERATOR CONTAINING THE FULLERENE**

(30) Priority: 31.03.2004 JP 2004102956
(71) Applicant: Tabata, Yasuhiko, Uji-shi, Kyoto 611-0024 (JP); NIPPON KAYAKU KABUSHIKI KAISHA, Tokyo 102-8172 (JP)
(72) Inventor: TABATA, Yasuhiko, 6110024 (JP); YAMADA, Masatoshi, Kyoto-shi, Kyoto 6068203 (JP); MASUDA, Akira, 3380001 (JP)
(74) Representative: Gille Hrabal Struck Neidlein Prop Roos
(86) International application number: PCT/JP2005/006271
(87) International publication number: WO 2005/095494

(57) **Abstract**

A water-soluble fullerene wherein the number of water-soluble polymers bonded has been regulated can be obtained by coupling water-soluble polymers with a fullerene having functional groups in its molecule via the functional groups. This water-soluble fullerene can be used in the photodynamic therapy or supersonic therapy of cancer through the use thereof as an active oxygen generator.

## Description

### TECHNICAL FIELD

The present invention relates to a novel water-soluble fullerene, a process for producing the same and an active oxygen generator containing the fullerene. More specifically, it relates to a novel water-soluble fullerene obtained by linking a water-soluble polymer to a fullerene having a functional group in the molecule through the functional group, a process for producing the same and an active oxygen generator containing the fullerene.

### BACKGROUND ART

Active oxygen such as singlet oxygen or superoxide anion can be generated by irradiating visible light, etc. to various active oxygen generators such as fullerenes and porphyrin derivatives. This active oxygen is highly reactive and exhibits cytotoxicities such as cleavage of DNA in a cell, suppression of cell growth, inhibition of proteolytic enzymes activity, and therefore, for example, its effects are expected on various diseases such as carcinoma, virus infection, intracellular parasitic infection, pulmonary fibrosis, liver cirrhosis, chronic nephritis, arterial sclerosis, diabetic retinopathy, senile macular degeneration and vasoconstriction lesion.

As for fullerene which is one of active oxygen generators, there have been known compounds such as pure carbon substances such as C₆₀ and C₇₀ depending on the number of n and carbon clusters which contain a metal or metal oxide (Non-Patent Document 1). Since fullerene in itself is water-insoluble, it is difficult to administer it into a living body. In the meantime, macromolecular materials more easily migrate to and tend to stay for a prolonged time in cancer tissues in comparison with normal tissues due to the difference in tissue structure (Non-Patent Document 2). For these reasons, it has been studied to link various water-soluble polymers to fullerene so as to obtain water-solubility as well as properties to specifically migrate to cancer tissues and retain there and thereby alleviate side effects caused by cytotoxicity of active oxygen on normal tissues. For such a water-soluble polymer, polyethylene glycol, polyvinyl alcohol, dextran, pullulan, starch and derivatives of these polymers have been suggested (Non-Patent Document 3, Patent Document 1).
In addition, it is known that the number of substituents linked to fullerene does affect significantly on the amount of active oxygen generated (Non-Patent Document 4).

Photosensitizers accumulated in cancer tissues generate singlet oxygen with high reactivity by light irradiation and selectively destroy only cancer tissues around them. Such a cancer therapy which combines photosensitizers with light irradiation is referred to as photodynamic therapy. As a photosensitizer for this photodynamic therapy, a fullerene to which are directly linked a plurality of polyethylene glycols having a methyl group at one end and an amino group at the other end is known (Patent Document 1).

Furthermore, when an ultrasonic wave is irradiated to liquid, bubbles are generated in the liquid (cavitation), and heat and pressure are locally generated when these bubbles collapse. This causes radicals (·OH, etc.) and these radicals emit light mainly in a wavelength range of 300-600 nm when they recombine or transit from an excited state to ground state. This phenomenon is known as sonoluminescence (Non-Patent Document 5). An active oxygen generator for sonodynamic therapy is known which contains a fullerene to which a plurality of polyethylene glycols having a methyl group at one end and an amino group at the other end are directly linked using this (Patent Document 2).
Patent Document 1: JP-A-9-235235
Patent Document 2: JP-A-2002-241307
Non-Patent Document 1: Kagaku (Chemistry), 50 (6), 1995
Non-Patent Document 2: Matsumura et al., Gan to Kagakuryohou (Japanese Journal of Cancer and Chemotherapy), Vol. 14, No. 3, p821-829, 1987
Non-Patent Document 3: BIOINDUSTRY, Vol. 14, No. 7, p30-37, 1997
Non-Patent Document 4: Toxicology in vitro, Vol. 16, p41-46, 2002
Non-Patent Document 5: Science, Vol. 247, p1439-1445,1990

### DISCLOSURE OF THE INVENTION

Although fullerenes as mentioned above to which plural water-soluble polymers such as polyethylene glycols are linked have been obtained, the number of the linked polyethylene glycols is not constant. That is, the number of the linked water-soluble polymers cannot be controlled and accordingly there occurs variation in the amount of generated active oxygen, which depends on the number of the bindings, and standardization of a product is difficult when it is intended to be applied for medical use.
An object of the present invention is to provide a water-soluble fullerene controlled in the number of linked water-soluble polymers, that is, a water-soluble fullerene linked to water-soluble polymers controlled in the number of modifying molecules. Besides, another object of the present invention is to provide a process for producing such a water-soluble fullerene and an active oxygen generator containing the same.

The present invention relates to a water-soluble fullerene wherein the fullerene has a functional group in the molecule and a water-soluble polymer is linked through the functional group.
Furthermore, the present invention relates to a process for producing a water-soluble fullerene characterized by reacting a water-soluble polymer with a functional group of a fullerene having the functional group in a molecule.
Furthermore, the present invention relates to an active oxygen generator containing a water-soluble fullerene mentioned above or a water-soluble fullerene produced by the above-mentioned production process.

According to the present invention, a water-soluble fullerene controlled in the number of linked water-soluble polymers can be obtained. When a light is irradiated to a water-soluble fullerene of the present invention, O₂⁻ (superoxide anion) is generated in a wide wavelength range from 220 nm to visible light area (380-780 nm). In particular, it exhibits high O₂⁻ generating ability in a wavelength range of 260-450 nm and therefore it can be applied to photodynamic therapy of cancer. In addition, since the light generated by sonoluminescence caused by ultrasonic irradiation mainly has a wavelength range of 300-600 nm, the water-soluble fullerene of the present invention generates O₂⁻ by this. The water-soluble fullerene of the present invention is suitable for sonodynamic therapy.

### BRIEF DESCRIPTION OF THE DRAWINGS

Fig. 1 shows the results of measuring a molecular weight by subjecting one molecule PEG-linked fullerene obtained in Example 1 to high performance liquid chromatography;
Fig. 2 shows the results of measuring a molecular weight by subjecting fullerene-water-soluble polymer conjugate synthesized according to a method described in Example 1 of JP-A-9-235235 to high performance liquid chromatography;
Fig. 3 shows the results of measuring a particle diameter of the water-soluble fullerenes of the present invention obtained in Example 1 and Example 2 by light scattering method;
Fig. 4 shows the results of measuring an amount of active oxygen generated by light irradiation of the water-soluble fullerene of the present invention obtained in Example 2;
Fig. 5 shows the results of measuring in vitro cancer cell growth inhibitory activity by light irradiation of the water-soluble fullerene of the present invention obtained in Example 2;
Fig. 6 shows the results of measuring in vivo anticancer activity by light irradiation of the water-soluble fullerene of the present invention obtained in Example 1;
Fig. 7 shows the results of measuring active oxygen abundance by ultrasonic irradiation of the water-soluble fullerene of the present invention obtained in Example 2; and
Fig. 8 shows the results of measuring in vitro cancer cell growth inhibitory activity by ultrasonic irradiation of the water-soluble fullerene of the present invention obtained in Example 1.

### BEST MODE FOR CARRYING OUT THE INVENTION

The water-soluble fullerene of the present invention is characterized in that a water-soluble polymer is linked to a fullerene having a functional group in the molecule through the functional group.
The type of fullerene to be used in the present invention is not limited in particular and any type of fullerene which generates active oxygen can be used. Specifically, C₆₀ fullerene, which is a pure carbon substance having 60 carbon atoms, C₇₀ fullerene, nanotube fullerenes which are pure carbon substances, various higher fullerenes can be used. These various fullerenes are commercially available and, for example, can be obtained from Honjo Chemical Co., Ltd., Mitsubishi Corporation, Tokyo Kasei Kogyo Co., Ltd., etc. (product name: C₆₀ fullerene, C₇₀ fullerene, multi-wall nanotube, single wall nanotube, etc.). Above all, it is preferable to use C₆₀ fullerene from the viewpoint of supply and easiness of handling.

Examples of the functional group linked to fullerene include a carboxyl group, an amino group, a hydroxyl group, a cyano group and a thiol group. The number of the bindings is preferably 1 to 5, and more preferably 1. Particularly preferable is a fullerene having one carboxyl group in the molecule. Such a fullerene having one carboxyl group in the molecule is commercially available and, for example, can be obtained from reagent companies such as Science Laboratories Co., Ltd. In addition, it can be synthesized by a method described in a document "Tetrahedron Letters vol. 36, No. 38, p.6,843, 1995".

A water-soluble polymer to be used in the present invention is not limited in particular, but those having a molecular weight of 1,000-1,000,000, preferably 4,000-50,000 can be used.

A water-soluble polymer to be used in the present invention is not limited in particular, and various commercially available water-soluble polymers can be used. Above all, nonionic water-solubility synthetic polymers such as polyethylene glycol, polypropylene glycol, polyvinyl alcohol, polyvinylpyrrolidone; dextran; pullulan; ionic or nonionic polysaccharides such as starch, hydroxyethylated starch and hydroxypropyl starch; modified substances thereof; copolymer or composite of two or three ingredients of these water-soluble polymers; hyaluronic acid, chitosan, chitinous derivatives, etc. can be used. Above all, polyethylene glycol which has a solvent commonly usable with fullerene, a functional group participating in the linking reaction with fullerene only at the molecular end and a simple chemical bonding style can be preferably used. Particularly it is preferable to use 4,000 to 15,000 polyethylene glycol.

As water-soluble polymers to be used in the present invention, those having a reactive group which can react with a functional group of fullerene can be normally used. Examples of the reactive group include a carboxyl group, an amino group, a hydroxyl group, a cyano group and a thiol group. Above all, a reactive group having dehydration condensation reactivity such as an amino group, a hydroxyl group is preferable, and more preferably it is an amino group. Here, the reactive group may be linked to a water-soluble polymer through a C1-C6 alkyl group. Such a reactive group may be located at any position in the molecule of a water-soluble polymer as long as the position is suitable for linking to a fullerene but it is preferably located at the end of the water-soluble polymer in consideration of facility of linking. When a water-soluble polymer which does not have such a reactive group is used, it is necessary to first introduce a reactive group before linking to fullerene.

As water-soluble polymers to be used in the present invention, those having an inactive group at one end and a reactive group at the other end are preferable. Examples of the inactive group include a C1-C6 alkyl group, a C1-C6 alkoxy group, a benzyl group and the other groups normally used as a protecting group. When polyethylene glycol is used as a water-soluble polymer, a C1-C6 alkyl group is preferable. Examples of a C1-C6 alkyl group include a methyl group, an ethyl group, an n-propyl group, an isopropyl group, an n-butyl group, a sec-butyl group, a tert-butyl group, an n-pentyl group, an n-hexyl group, and a methyl group is preferable from availability.

As water-soluble polymers to be used in the present invention, polyethylene glycol having an inactive group at one end and a reactive group at the other end and having a molecular weight of 4000 to 15000 are preferable, and particularly, polyethylene glycol having a C1-C6 alkyl group at one end and an amino group at the other end and having a molecular weight of 4000 to 15000 are preferable. Composite of polyethylene glycol having an inactive group at one end and having a molecular weight of 4000 to 15000 and a compound having a reactive group which can react with a functional group of a fullerene is also preferable and particularly, a reaction product of polyethylene glycol having a C1-C6 alkyl group at one end and an amino group at the other end and having a molecular weight of 4000 to 15000 and an amino acid such as asparagic acid is preferable.

Water-soluble fullerene of the present invention only has to be provided with water-solubility which allows administration to a living body. The fullerene linked to a water-soluble polymer preferably forms aggregate and the size of the aggregate is preferably around 20 to 400 nm and more preferably around 30 to 200 nm in the measurement by light scattering method in consideration of facility of transition to and accumulation at tissues such as cancer and transition to normal cells. The molecular weight of the water-soluble polymer which is necessary to form such an aggregate varies depending on the type of the water-soluble polymer used and the number of them linked to fullerene. For example, molecular weight of 2,000 to 30,000 is preferable in the case of polyethylene glycol having one amino group per molecule, and it is preferably in a range of 4,000 to 15,000.

The process for producing a water-soluble fullerene of the present invention is characterized by reacting a water-soluble polymer with a functional group of a fullerene having the functional group in the molecule. The number of the linked water-soluble polymers can be controlled by using a fullerene having a functional group in the molecule. For example, when fullerene having one functional group in the molecule is used, fullerene which has one water-soluble polymer is obtained, and when fullerene having two functional groups in the molecule is used, a fullerene which has two water-soluble polymers is obtained. For example, in the case that a fullerene having a carboxyl group in the molecule and a water-soluble polymer having a C1-C6 alkyl group at one end and a reactive group at the other end are subjected to condensation reaction, the ratio of the fullerene having a carboxyl group in the molecule and the water-soluble polymer having a C1-C6 alkyl group at one end and a reactive group at the other end to be used is around 0.1 to 10 mol of the latter to 1 mol of the former, preferably around 2 mol of the latter to 0.5 mol of the former, and more preferably it is around 1 to 1.1 mol of the latter to 1 mol of the former.

Examples of the reaction include well-known reactions which generate a chemical bond such as condensation reaction, addition reaction, substitution reaction. For example, in the case of condensation reaction, when the reactive group of water-soluble polymer is an amino group, the reaction is performed by conventional peptide condensation reaction. In the case of peptide condensation reaction, the functional group linked to a fullerene is a carboxyl group, and it is performed in the presence of a dehydration condensing agent. The dehydration condensing agent includes carbodiimides such as dicyclohexylcarbodiimide, diisopropylcarbodiimide, 1-dimethylaminopropyl-3-ethylcarbodiimide, phosphonium salts such as benzotriazol-1-yl-tris(dimethylamino)phosphonium hexafluorophosphate, diphenylphosphoryl azide, and preferably it is diisopropylcarbodiimide. The amount of the dehydration condensing agent to be used is 0.5 to 10 mol equivalent for a carboxyl group of fullerene, and preferably it is 1 to 2 mol equivalent. The reaction is performed in the presence of or absence of an additive, and the additive includes N-hydroxysuccinimide, 1-hydroxybenzotriazole, 4-nitrophenol, pentafluorophenol, and preferably it is 1-hydroxybenzotriazole. The amount of the additive to be used is 0.5 to 10 mol equivalent for a carboxyl group of fullerene, and preferably it is 1 to 2 mol equivalent.

An organic solvent is used in this reaction. The organic solvent is not limited in particular as far as the reaction proceeds and examples thereof include aromatic hydrocarbons such as benzene, toluene, xylene, halogenated hydrocarbons such as methylene chloride, chloroform, 1,2-dichloroethane, chlorobenzene, bromobenzene, 1,2-dichlorobenzene; ethers such as diethyl ether, diisopropyl ether, tetrahydrofuran, dioxane, dimethoxyethane, diethylene glycol dimethyl ether; nitriles such as acetonitrile, propionitrile; amides such as dimethylformamide, dimethylacetamide, hexamethylphosphoric triamide; urea such as N,N-dimethylimidazolidinone; and mixed solvents of these solvents, and preferably it is toluene, chlorobenzene, bromobenzene, 1,2-dichlorobenzene and 1:1 mixed solvent of dioxane and dichloromethane, and more preferably it is bromobenzene. The reaction temperature is -20 to 100°C, preferably 0 to 50°C, and more preferably room temperature to 37°C, and the reaction time is 1 to 72 hours and preferably 3 to 24 hours. It is preferable to perform this reaction under light shielding. The obtained reaction product can be isolated and purified by separation means known per se, for example, by vacuum concentration, solvent extraction, crystallization, chromatography, dialysis, freeze-dry, etc.

The water-soluble polymer is not limited in particular, and various commercially available water-soluble polymers mentioned above can be used. Here, as for the water-soluble polymer, usually those having a reactive group such as amino group as stated above is used so as to enable it to link to fullerene. When a water-soluble polymer which does not have reactive group is used, it is necessary to first introduce a reactive group before linking to fullerene. For example, in a water-soluble polymer having a carboxyl group, an amino group is introduced into a polymer chain by a binding reaction of a carboxyl group and an amino compound using N-hydroxysuccinimide and carbodiimide, carbodiimide or ethyl chlorocarbonate. For example, in order to introduce an amino group into polyethylene glycol, polyethylene glycol having a carboxyl group at one end is dissolved in a phosphate buffer of pH 5.0 (10% by weight), and a water-soluble carbodiimide is added in 3-time molar amount for a carboxyl group and the carboxyl group is activated by performing agitation for 1 hour at room temperature. Then, 10-time molar amount of ethylene diamine for a carboxyl group is added and allowed to react at room temperature for 6 hours. A polyethylene glycol to which an amino group has been introduced at one end can be obtained by dialyzing the obtained reaction liquid with water. A polyethylene glycol in which one end is an aminopropyl group and the other end is a methyl group, that is, CH₃(OCH₂CH₂)ₙO(CH₂)₃NH₂ (n represents an integer of around 90-300) is also available from Nippon Oil & Fats Co., Ltd.

As mentioned above, the water-soluble polymer may also be a composite of a polyethylene glycol having an inactive group at one end and a compound having a reactive group which reacts with a functional group of fullerene, and the compound having a reactive group which reacts with a functional group of fullerene includes an amino acid, and preferably an acidic amino acid such as asparagic acid and glutamic acid. The composite is produced, for example, as follows. An acidic amino acid having a protected amino group and a polyethylene glycol having a C1-C6 alkyl group at one end and a C1-C6 alkyl group substituted with an amino group at the other end are reacted in the presence of a dehydration condensing agent in an organic solvent. The reaction conditions may be a similar condition as in the case of peptide condensation reaction in which the reactive group of the above-mentioned water-soluble polymer is an amino group and the functional group of fullerene is a carboxyl group. A water-soluble polymer which is an amino acid derivative linked to a polyethylene glycol having a C1-C6 alkyl group at one end can be obtained by subjecting the resulted reactant to deprotection reaction according to the protecting group. Specifically, it is shown in Example 3 described below.

The active oxygen generator of the present invention contains a water-soluble fullerene linked to a water-soluble polymer obtained as above, and it is used as an aqueous solution or a solution of a water-containing solvent. When this water-soluble fullerene is irradiated with light, O₂⁻ generates in a wide wavelength range from 220 nm to visible light area (380-780 nm). In particular, it shows high O₂⁻ generating ability in a wavelength range of 260-450 nm. Therefore, it can be applied to photodynamic therapy of cancer by light irradiation. In addition, since the light generated by sonoluminescence caused by ultrasonic irradiation mainly has a wavelength range of 300 to 600 nm, the active oxygen generator of the present invention is suitable for sonodynamic therapy.
Singlet oxygen (¹O₂), superoxide anion (O₂⁻), a hydrogen peroxide (H₂O₂), hydroxy radical (-OH) are included in the active oxygen generated by the active oxygen generator of the present invention.

The water-soluble fullerene of the present invention forms aggregate of a certain size in an aqueous solvent. Examples of the aqueous solvent include water and water-acetonitrile. Since the active oxygen generator of the present invention is a fullerene linked to a water-soluble polymer, it has enough water-solubility so as to be administered to a living body and in addition, since it forms aggregate of a certain size, it is supposed to have high migration to and retaining properties at cancer tissues and inflammatory tissues. It is supposed that this aggregate is an aggregate of fullerene which keeps the number of the linked water-soluble polymers and takes a polymer micelle structure.

Since the fullerene contained in the active oxygen generator of the present invention exhibits cytotoxicity by generating active oxygen such as singlet oxygen or superoxide anion in an aqueous solvent by the light generated by sonoluminescence caused by ultrasonic irradiation, it can be used for treatment of various diseases containing cancer as described below. As the ultrasonic wave to be irradiated, frequency of about 100 KHz to 20 MHz, in particular about 1 to 3 MHz can be preferably used. Irradiation is preferably performed at output of about 0.1 to 5 Watt/cm², particularly about 2 Watt/cm². The irradiation time varies depending on the frequencies used, irradiation output, but it is about 5 to 300 seconds, and preferably it is about 30 to 120 seconds, and in the case of pulse irradiation, the dutycycle thereof is about 1 to 100%, preferably about 10%.

The active oxygen generator of the present invention is effective in the treatment of any type of cancer for which active oxygen shows cytotoxicity, virus infection, intracellular parasitism infection, pulmonary fibrosis, liver cirrhosis, chronic nephritis, arterial sclerosis, diabetic retinopathy, senile macular degeneration and vasoconstriction lesions, etc. Examples of cancer include every solid cancer occurring in the surface and the inside of organs such as lung cancer, hepatic carcinoma, pancreatic carcinoma, stomach intestinal cancer, bladder cancer, renal cancer and brain tumor. Above all, when the active oxygen generator of the present invention is used for sonodynamic therapy, it can be effectively used for the treatment of cancers deep in the body to which light irradiation is impossible and photodynamic therapy of cancer is impossible conventionally. As for the other disease, since the lesion or infected cells or affected cells are located in the internal parts of organs, they can be treated by accumulating the active oxygen generator by a method suitable for the site and then irradiating light or ultrasonic from the outside.

The active oxygen generator of the present invention can be made into any pharmaceutical form such as injection agent, dispersion agent, liquid agent, solid powder. For example, when it is provided as an injection agent, the active oxygen generator of the present invention can be combined with various additives such as buffer, physiological saline, preservative, distilled water for injection commonly used for injection agent to form an injection agent. The active oxygen generator of the present invention can be intravenously, intra-arterially, intramuscularly, subcutaneously or intradermally administered, and the dosage varies depending on the administration route, age and sex of the patient, type and condition of the disease, but it can be administered once to dividedly several times a day per adult in about 1 to 10 mg/kg in terms of water-soluble fullerene of the present invention.

As stated above, polymer material is easy to migrate to and accumulate at cancer tissues and inflammatory tissues in comparison with normal tissues. Therefore, when the active oxygen generator of the present invention containing fullerene linked to a water-soluble polymer is administered to a living body, it accumulates in cancer tissues and inflammatory tissues compared with normal tissues, and it retains in cancer tissues and inflammatory tissues for a long time in a higher concentration compared with normal tissues. On the other hand, since the active oxygen generator of the present invention is excreted more rapidly from normal tissues than from cancer tissues and inflammatory tissues, the concentration of the active oxygen generator of the present invention in cancer tissues and inflammatory tissues in some length of time after it is administered to a living body is significantly higher than the concentration in normal tissues, and the active oxygen generator of the present invention will be specifically distributed in cancer tissues and inflammatory tissues in high concentration. Therefore, if light or ultrasonic is irradiated to the living body, in a while after the active oxygen generator of the present invention is administered to a living body, the active oxygen generator generates active oxygen such as singlet oxygen by light or light generated by sonoluminescence caused by ultrasonic irradiation, and exhibits anticancer activity and antiinflammatory activity specifically in cancer tissues and inflammatory tissues. On the other hand, since the concentration of the active oxygen generator of the present invention is low in normal tissues, the cytotoxicity in normal tissues is not no high as compared in cancer tissues and inflammatory tissues and therefore, it is expected that side effects in normal tissues will be alleviated.

The length of time duirng which the concentration of the active oxygen generator of the present invention in cancer tissues and inflammatory tissues becomes significantly higher than the concentration in normal tissues after it is administered to a human body and light or ultrasonic irradiation becomes possible varies depending on the metabolic condition in the site to be treated of an individual patient and time-course change of distribution of active oxygen generator but generally it is preferable to conduct light or ultrasonic irradiation in about 0.1 to 48 hours, particularly about 24 hours after administration. When an ultrasonic wave is irradiated to human, the ultrasonic wave having a frequency mentioned above is irradiated at an output and for a length of time as mentioned above. Therefore, in order to treat a patient using the active oxygen generator of the present invention, the active oxygen generator of the present invention is administered, for example, in a pharmaceutical form of injection agent, and irradiation is performed with a light irradiation equipment or ultrasonic generating equipment in about 0.1 to 48 hours. Dosage and frequency of administration/irradiation, administration times can be determined in accordance with age, body weight, sex of a patient, type and conditions of the disease.

The active oxygen generator of the present invention does not specifically migrate to and accumulate at cancer tissues and inflammatory tissues, but it is possible to have the cytotoxicity exhibited in a target tissue or cell by using any method to delivery the active oxygen generator of the present invention to the target tissue or cell, for example, by using a specifically delivering method with a drug delivery system. Such a method includes a method of injecting the active oxygen generator of the the present invention directly to the target tissue or cell (delivering to most parts within the body is possible by, for example, using endoscope) and a method of administering the active oxygen generator of the the present invention linked to a cell recognition factor such as antibody, lectin, cell adhering factor and sugar chain to the target tissue or cell. In addition, it is also possible to generate active oxygen only at desired points to exhibit cytotoxicity by irradiating light or ultrasonic only at points where generation of active oxygen is desired after the active oxygen generator of the the present invention is administered to a living body. Furthermore, selectivity of the region where cytotoxicity is exhibited can be improved by focusing the light or ultrasonic wave.

Hereinbelow, the present invention is described more in detail with reference to Examples, Referential Examples and Test Examples but the scope of the present invention is not limited to these.
Referential Example 1
Synthesis of (1,2-methano [60] fullerene)-61-carboxylic acid
tert-Butyl ester of (1,2-methano[60]fullerene)-61-carboxylic acid obtained by a method described in Tetrahedron Letters vol.36, No.38, p.6843, 1995 (540 mg, 0.65 mmol) was dissolved in toluene (380 mL), added with 4-toluenesulfonic acid monohydrate (222 mg, 1.17 mmol) and heated for ten hours under reflux. The deposited brown precipitate was filtered and sequentially washed with toluene, distilled water and ethanol and dried under reduced pressure and (1,2-methano[60]fullerene)-61-carboxylic acid (338 mg, yield 67%) was obtained as a brown crystal.

FAB-MS (positive mode): m/z779 (M+H)⁺;
¹H-NMR (CDCl₃/DMSO-d₆ (1:1), ppm): 5.15 (¹H, s).

### Example 1

### Synthesis of fullerene linked to one molecule of PEG (molecular weight 5000)

14.7 mL of 0.33 mM bromobenzene solution of (1,2-methano[60]fullerene)-61-carboxylic acid was added to 2 mL of bromobenzene solution containing a molar equvalent of polyethylene glycol having a methyl group at one end and an aminopropyl group at the other end (PEG, molecular weight: 5000, product of Nippon Oil & Fats), adding two molar equvalents of 1-hydroxybenzotriazole and N,N'-diisopropylcarbodiimide, and stirred at room temperature for 24 hours under light shielding condition. The reaction liquid was extracted with the same amount of distilled water. The aqueous layer was passed through a cation exchange resin column (SP-Toyopearl 650 M, H⁺-type) and then the effluent was freeze-dried and fullerene linked to one molecule of PEG (molecular weight 5000) (24.4 mg) was obtained.
Thin-layer chromatography (eluent: 20% metanol-dichloromethane) relative mobility (Rf): 0.75.

### Example 2

### Synthesis of fullerene linked to one molecule of PEG (molecular weight 12000)

PEG having a methyl group at one end and an aminopropyl group at the other end (product of Nippon Oil & Fats) having a molecular weight of 12000 in stead of molecular weight of 5000 was used and the same procedure was conducted as in Example 1 and fullerene linked to one molecule of PEG (molecular weight 12000) (47.4 mg) was obtained.
Thin-layer chromatography (eluent: 20% methanol-dichloromethane) relative mobility (Rf): 0.73.

### Test Example 1

### Analysis of molecular weight of fullerene linked to one molecule of PEG (molecular weight 5000) synthesized in

### Example 1

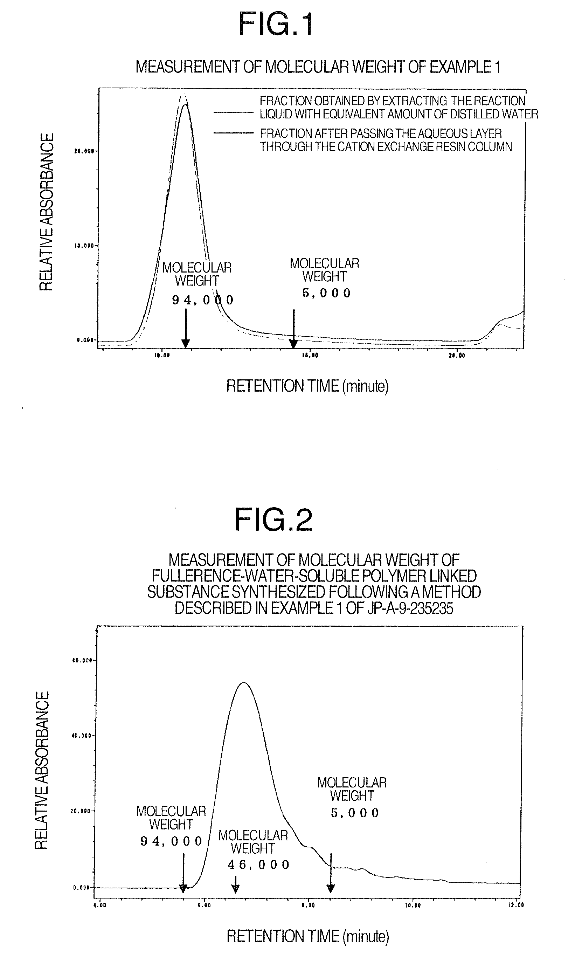

Measurement of molecular weight of a fraction obtained by extracting the reaction liquid with equivalent amount of distilled water in Example 1 and a fraction after passing the aqueous layer through the cation exchange resin column was carried out using high performance liquid chromatography system 8020 (Tosoh Co., Ltd.) with TSKgel G3000PW_{XL} (Tosoh Co., Ltd.). 50 mM phosphate buffer (pH 6.9) containing 20% acetonitrile and 0.3 M sodium chloride was used as mobile phase with a flow rate of mobile phase of 0.5 mL/min and the detection was performed at ultra-violet absorption of fullerene. The results are shown in Fig. 1. Polyethylene glycols having known molecular weight (molecular weight 94,000 and 5,000) were used as molecular weight markers and the retention time was shown in Fig. 1.

As a comparative control, PEG having a methyl group at one end and an aminopropyl group at the other end and having a molecular weight 5000 was used, and the measurement of molecular weight of a fullerene-water-soluble polymer conjugate synthesized in a molar ratio of 1:10 following a method described in Example 1 of JP-A-09-235235 was carried out. 50 mM phosphate buffer (pH 6.9) containing 0.3 M sodium chloride was used as mobile phase with a flow rate of mobile phase of 1 mL/min and the detection was performed at ultra-violet absorption of fullerene. The results are shown in Fig. 2. Polyethylene glycols having known molecular weight (molecular weight 94,000, 46,000 and 5,000) were used as molecular weight markers and the retention time was shown in Fig. 2.

From these results, the compound of Example 1 having a molecular weight of about 5,800 formed aggregate having a molecular weight of about 100,000 by self assembly, and it was shown that the size became larger. In addition, the aggregate was formed with a good reproducibility even in a condition in which the solution composition was different, and showed a small single peak of molecular weight distribution because the number of linked water-soluble polymers was constant. On the other hand, the fullerene-water-soluble polymer conjugate synthesized following a method described in Example 1 of JP-A-09-235235 had major component lower than the molecular weight of 46,000 but the molecular weight distribution was wide and plural peaks were observed, and thus it has been shown that uniform aggregate can be obtained by controlling the number of the linked water-soluble polymers.

It is known that the number of substituents linked to fullerene greatly influences amount of generation of active oxygen (document: Toxicology in vitro, Vol. 16, p41-46,2002), and being a derivative having one substituent like a compound of Example 1 is useful from a viewpoint of targeting of drug delivery system and generation amount of active oxygen as compared with conventional fullerene water-soluble polymer conjugate.

### Test Example 2

### Measurement of particle size of fullerene linked to one molecule of PEG (molecular weight 5000) synthesized in

### Example 1

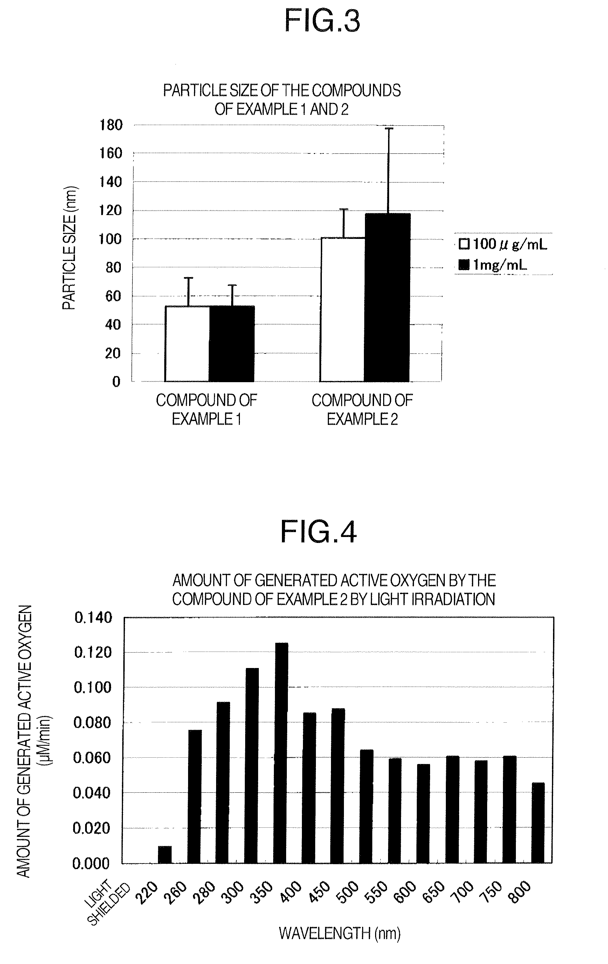

Particle size measurement by light scattering method was performed on the water-soluble fullerene of the present invention. The water-soluble fullerene which was synthesized with Example 1 was dissolved in distilled water so that the final concentration might be 1 mg/mL and 100 µg/mL. This solution was measured with light scattering measuring apparatus DLS-7000 (Otsuka Electronics Co., Ltd.). The results of measurement are shown in Fig. 3.
The results of the measurement showed that the compound of Example 1 was aggregate which had particle diameters of about 50 nm which particle diameters were relatively uniform and that the compound of Example 2 was aggregate which had particle diameters of about 100 nm which particle diameters distributes in a little wide range, respectively. These particles diameters are considered to be large enough to exhitit EPR effect (Enhanced Permeation and Retention effect) that polymer materials are easy to migrate to cancer tissues and tends to retain in cancer tissues for a long time in comparison with normal tissues.

### Test Example 3

### Measurement of amount of active oxygen generated by fullerene linked to one molecule of PEG (molecular weight 12000) synthesized in Example 2

The amount of generated active oxygen (superoxide anion, O₂⁻) was measured by cytochrome method. 200 µL of a solution in which cytochrome c (Nacalai Tesque Corporation) was dissolved in a Hanks' balanced salt solution (HBSS, pH 7.4, Lifetech Oriental Company) so that the final concentration might be 50 µM and 200 µL of a solution in which fullerene linked to one molecule of PEG (molecular weight 12000) prepared in Example 2 was dissolved in HBSS so that the final concentration might be 200 µM were mixed. This mixed solution was irradiated with light of various wavelengths (220-800 nm) with spectrophotofluorometer F-2000 (Hitachi, Ltd.) at 30°C for 20 minutes. After irradiation, absorbance of the solution at 550 nm was measured with spectrophotometer DU-650 (Beckmann Company). The amount of generated O₂⁻ per minute was shown in Fig. 4 assuming a solution under light shielding condition at 30°C for 20 minutes as control.

When the water-soluble fullerene of Example 2 is irradiated with light, generation of O₂⁻ was observed in a wide wavelength range from ultraviolet to visible light areas, and the generation which was particularly significant was recognized in a wide wavelength range of 260 to 450 nm. Therefore, it has been demonstrated to be able to be applied to photodynamic therapy of cancer by light irradiation. In addition, since the light by sonoluminescence caused by ultrasonic irradiation mainly had a wavelength range of 300 to 600 nm, it has been demonstrated that the compound of the present invention was suitable for sonodynamic therapy.

### Example 3

### Synthesis of water-soluble fullerene in which fullerene is linked to water-soluble polymer in which two PEG (molecular weight 5000) molecules are amide bonded with carboxyl groups of L-asparagic acid

The same procedure was carried out as in Example 1 and water-soluble fullerene was obtained in which carboxyl groups of fullerene are linked to water-soluble polymer in which two PEG molecules each having a methyl group at one end and an aminopropyl group at the other end (molecular weight 5000) are amide bonded with two carboxyl groups of L-asparagic acid.
5 mL of N,N-dimethylformamide solution of 50 mM Boc-L-asparagic acid (product of Wako Pure Chemical) was added to 10 mL of N,N-dimethylformamide solution containing 3-time molar amount of polyethylene glycol (PEG, molecular weight: 5000, product of Nippon Oil & Fats) having a methyl group at one end and an aminopropyl group at the other end, adding 3-time molar amount of 1-hydroxybenzotriazole and N,N'-diisopropylcarbodiimide, and stirred at room temperature for 24 hours under light shielding condition. Diisopropyl ether was added to the reaction liquid and a precipitation was obtained. After the precipitation was dissolved in distilled water, it was passed through an anion exchange resin column (DEAE-TOYOPEARL 650M, Cl⁻-type) and a cation exchange resin column (SP-TOYOPEARL 650M, H⁺-type), and the effluent was freeze-dried and Boc-L-asparagic acid amide bonded with two molecules of PEG (molecular weight 5000) at carboxyl groups was obtained. 1 g of the obtained compound was dissolved in 5 mL of trifluoroacetic acid and deprotection was performed at room temperature for one hour. Diisopropyl ether was added to the reaction liquid, and L-asparagic acid amide bonded with two molecules of PEG (molecular weight 5000) at carboxyl groups was obtained by drying the precipitation under reduced pressure.
L-asparagic acid amide bonded with two molecules of PEG (molecular weight 5000) at carboxyl groups was used instead of polyethylene glycol having a methyl group at one end and an aminopropyl group at the other end and having a molecular weight of 5000 in Example 1, and the same procedure was carried out as in Example 1 and fullerene linked to 2 PEG (molecular weight 5000) molecules was obtained.

### Test Example 4

### Measurement of inhibitory activity on cancer cell growth when water-soluble fullerene of Example 2 is irradiated with light

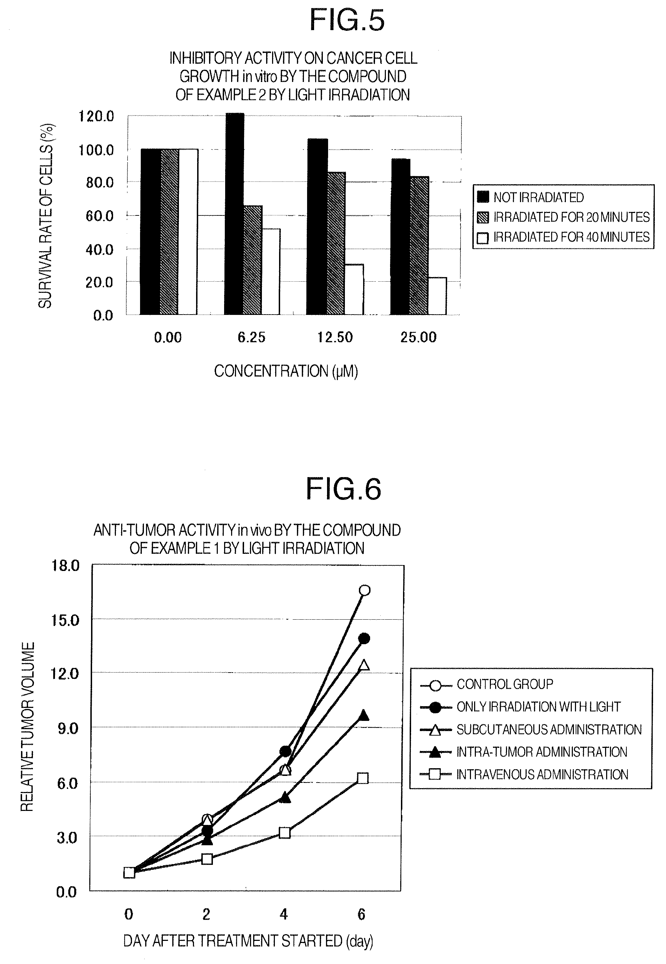

Cancer cell growth inhibitory activity in in vitro by light irradiation was measured. RLmale 1 cells (provided by Kyoto Pasteur Laboratory) were used as a cancer cell and these cells were incubated under culture conditions of 5% CO₂, 95% atmosphere, at 37°C in RPMI 1640 culture medium (Sigma Company, containing 10% fetal bovine serum) with 100 mm dish till they became 80% confluent. The water-soluble fullerene of Example 2 was dissolved in a culture medium of the same composition as used for culture of cancer cells under light shielding condition and after adjusted to the concentration of 250 µM, it was sterilized and filtered. The sterilized solution of 250 µM was diluted with a culture medium sequentially to prepare solutions of 125 µM and 62.5 µM. Various kinds of solutions thus prepared were dispensed into each well of 96-well plate by 10 µL and 10 µL of the culture medium which did not contain the compound of Example 2 was only dispensed in a control well. The cells which became 80% confluent mentioned above was prepared into a cell suspension of 5×10⁴ cell/mL and dispensed into each well mentioned above by 100 µL. After the 96 well plate was lightly stirred with a shaker, it was left in an 8W light box (Fuji Coor Sales) for 20 minutes or 40 minutes and irradiated with light. After the ight irradiaion, the plate was shaded with aluminum foil and cultured in an incubator (37°C, 5% CO₂) for three days. As for a control which was not irradiated with light, the plate was shaded with aluminum foil imediately after stirred and cultured for three days. After the culturing for three days, 10 µL each of viable count measurement reagent SF (Nacalai Tesque Corporation) was added into each well, and after kept warm in an incubator for 80 minutes, absorbance at 450 nm was measured with a microplate leader VERSAmax (Japanese Molecular Device Company). The survival rate of cancer cells was determined assuming that the absorbance at 450 nm for the case in which compound of Example 2 was not added was 100% survival rate and the results are shown in Fig. 5.
It has been demonstrated that the water-soluble fullerene of Example 2 significantly decreases the survival rate of cancer cells as the addition amount thereof increases when light is irradiated for 40 minutes.

### Test Example 5

### Measurement of anticancer activity of water-soluble fullerene of Example 1 when it is irradiated with light

Administration routes were changed and anticancer activity in vivo by light irradiation was compared. Cancer bearing mice were prepared by removing a tumor block which had been passaged from mouse colon cancer Colon26 cells (provided by Cancer Chemotherapy Center of Cancer Research Society) by subcutaneous transplantation of BALB/c-nn (female, Charles River Laboratories Japan, Inc.) and transplanting a strip piece onto the back subcutis of CDF1 mouse (female, Charles River Laboratories Japan, Inc.). The cancer bearing mice were used in the experiment in five days after transplantation in which cancer having a diameter of around 5 mm was formed in subcutis. The compound of Example 1 was dissolved in a phosphate-buffered physiological saline solution so that it might be 6 mg/mL and a group to which 100 µL of the solution was administered in the subcutaneous region of the tumor or into the tumor was irradiated at the tumor site with light using bluephase (Vivadent Company) in five minutes after administration. The irradiation condition was irradiation with output power of 650 W/cm² for four minutes using a probe having a diameter of 8 mm. This operation was performed for three conseutive days. For intravenous administration group, the compound of Example 1 was dissolved in a phosphate-buffered physiological saline solution so that it might be 60 mg/mL and and 100 µL of the solution was administered in tail vein of the mice and the tumor site was irradiated with light using bluephase in 24 hours after administration. The irradiation condition was the same as in the condition for subcutis intra-tumor administration group and this operation was performed for three conseutive days.
Cancer bearing mice to which water-soluble fullerene was not administered and which were not irradiated with light were used as a control group and the size of the tumor of the subcutaneous region was measured for each mouse with time. The cancer size was measured by actually measuring the major axis and the minor axis of the cancer with a slide caliper and calculated using a calculation formula reported by Winn (Natl.Cancer Inst Monogr., Vol. 2, p113-138 (1960)). The results are shown in Fig. 6.

Increase of a tumor volume was supressed when the administration of the water-soluble fullerene of Example 1 and light irradiation were combined, and the supression effect increased in the order of subcutaneous administration <intra-tumor administration < intravenous administration. Since the intravenous administration group exhibited the most effective resluts in spite of light irradiation after 24 hours, it has been shown that as for the water-soluble fullerene of the present invention, optimum injection method is administration through a blood vessel, which suggests the probability that EPR effect contributed.

### Test Example 6

### Measurement of inhibitory activity on cancer cell growth when the water-soluble fullerene of Example 2 is irradiated with an ultrasonic wave

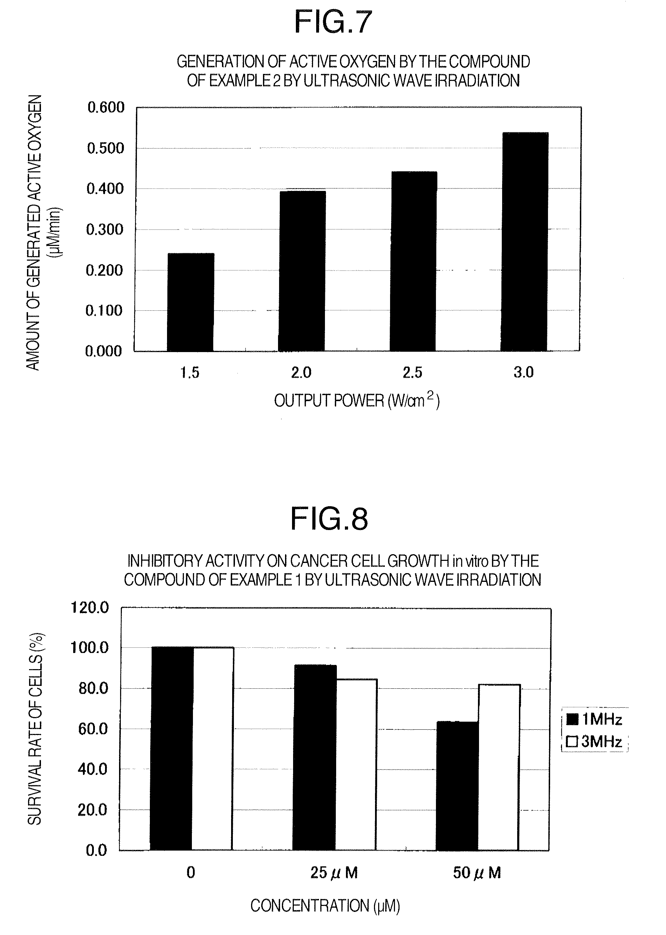

The amount of active oxygen (superoxide anion, O₂⁻) generated by ultrasonic irradiation was measured using viable count measurement reagent SF with reference to a report by Ukeda (DOJIN NEWS, No.96, p1-6,2000). A solution in which water-soluble fullerene of Example 2 was dissolved in a Hanks' balanced salt solution so that it might be 200 µM was dispensed to 35 mm dish by 750 µL. This was mixed with 600 µL of a Hanks' balanced salt solution and 150 µL of viable count measurement reagent SF and, while stirred, irradiated with ultrasonic waves of various output (1.5, 2.0, 2.5 and 3.0 W/cm²) with a frequency of 1 MHz, output mode (Duty cycle) 30% for five minutes from the liquid surface using an sonodynamic therapy device US-700 (Ito Ultrashort Wave Company). After irradiation, absorbance of the solution at 450 nm was measured with a spectrophotometer DU-650. The absorbance of the solution irradiated with ultrasonic wave without adding a compound at 450 nm was assumed as control and the amount of generated O₂⁻ per minute was shown in Fig. 7.
It has been demonstrated that when the water-soluble fullerene of Example 2 is irradiated with an ultrasonic wave, the amount of active oxygen increases according to the output increases.

### Test Example 7

### Measurement of inhibitory activity on cancer cell growth when the water-soluble fullerene of Example 1 is irradiated with an ultrasonic wave

Anticancer activity in vitro in ultrasonic irradiation was measured. RLmale1 cells were used in the similar way as in Test Example 4. The water-soluble fullerene of Example 1 was dissolved in a culture medium of the same composition as used for culture of cancer cells under light shielding condition and after adjusted to the concentration of 500 µM, it was sterilized and filtered. 250 µM solution was prepared from the sterilized 500 µM solution. The thus prepared solution was dispensed into each well of 6 well plate by 200 µL and 200 µL of the culture medium which did not contain the water-soluble fullerene of Example 1 was only dispensed in a control well. RLmale1 cells were adjusted to 1×10⁵ cell/mL and a cell suspension was obtained and dispensed into each well mentioned above by 2 mL. After the cells in the wells were lightly mixed with a pipet, ultrasonic waves having a frequency of 1 MHz or 3 MHz were irradated at an output power of 2.0 W/cm², output mode of 20% for two minutes from the base part of the plate through a conductive gel using an sonodynamic therapy apparatus US-700. After the ultrasonic irradiaion, the plate was shaded with aluminum foil and cultured in an incubator (37°C, 5% CO₂) for three days. As for a control which was not irradiated with ultrasonic wave, the plate was shaded with aluminum foil imediately after mixed and cultured for three days in the same way. After the culturing for three days, 100 µL each of a cell suspension was dispensed to 96-well plate from each well and 10 µL each of viable count measurement reagent SF was added into each well, and after kept warm in an incubator for 45 minutes, absorbance at 450 nm was measured with a microplate leader VERSAmax. The survival rate of cancer cells was determined assuming that the absorbance at 450 nm for the case in which water-soluble fullerene of Example 1 was not added was 100% survival rate and the results are shown in Fig. 8.

It has been demonstrated in ultrasonic irradiation that the survival rate of cancer cells decreases as the addition amount of the present invention compound increases in the same way as in Test Example 4. As for this effect, 1 MHz was more remarkable than the frequency of 3 MHz. Therefore, it has been shown that the water-soluble fullerene of the present invention can be used in sonodynamic therapy by ultrasonic irradiation as well as photodynamic therapy of cancer by light irradiation.

### INDUSTRIAL APPLICABILITY

As described above in detail, according to the present invention, water-soluble fullerene controlled in the number of linked water-soluble polymers can be obtained. When the water-soluble fullerene of the present invention is irradiated with light, O₂⁻ generates in a wide wavelength range from 220 nm to visible light area (380 to 780 nm). It shows high O₂⁻ generating ability in a wavelength range of 260 to 450 nm in particular. In addition, light generated by sonoluminescence caused by ultrasonic irradiation mainly has a wavelength range of 300 to 600 nm, and O₂⁻ generates when the water-soluble fullerene of the present invention is irradiated with an ultrasonic wave as well. Besides, the water-soluble fullerene of the present invention is highly accumulated in cancer tissues and inflammatory tissues. Therefore, the water-soluble fullerene of the present invention can be used as an active oxygen generator and can be appled to photodynamic therapy or sonodynamic therapy of cancer.

## Claims

1. A water-soluble fullerene wherein the fullerene has a functional group in the molecule and a water-soluble polymer is linked through the functional group.

2. The water-soluble fullerene according to claim 1 having 1 to 5 functional groups.

3. The water-soluble fullerene according to claim 1 or 2 having one functional group.

4. The water-soluble fullerene according to any of claims 1 to 3 wherein the functional group is a carboxyl group.

5. The water-soluble fullerene according to any of claims 1 to 4 wherein the fullerene is C₆₀ fullerene.

6. The water-soluble fullerene according to any of claims 1 to 5 wherein molecular weight of the water-soluble polymer is 1,000 to 1,000,000.

7. The water-soluble fullerene according to any of claims 1 to 6 wherein the water-soluble polymer is a water-soluble polymer selected from nonionic water-soluble synthetic polymers, nonionic or ionic polysaccharides, modified substances thereof, copolymer or composite of two or three ingredients of these water-soluble polymers, hyaluronic acid, chitosan and chitinous derivatives.

8. The water-soluble fullerene according to any of claims 1 to 7 wherein the water-soluble polymer is a water-soluble polymer having an inactive group at one end and a reactive group which reacts with a functional group of a fullerene at the other end.

9. The water-soluble fullerene according to claim 8 wherein the water-soluble polymer is a polyethylene glycol having an inactive group at one end and a reactive group which reacts with a functional group of a fullerene at the other end and having a molecular weight of 4000 to 15000.

10. The water-soluble fullerene according to claim 9 wherein the water-soluble polymer is a polyethylene glycol having a C1-C6 alkyl group at one end and a C1-6 alkyl group substituted with an amino group at the other end and having a molecular weight of 4000 to 15000.

11. The water-soluble fullerene according to claim 8 wherein the water-soluble polymer is a composite of a polyethylene glycol, having an inactive group at one end and having a molecular weight of 4000 to 15000, and a compound having a reactive group which reacts with a functional group of a fullerene.

12. The water-soluble fullerene according to claim 11 wherein the water-soluble polymer is a reaction product of a polyethylene glycol, having a C1-C6 alkyl group at one end and a C1-6 alkyl group substituted with an amino group at the other end, and an amino acid.

13. The water-soluble fullerene according to any of claims 1 to 12 wherein the water-soluble fullerene is in a form of aggregate.

14. The water-soluble fullerene according to claim 13 wherein the aggregate has a size of 20 to 400 nm.

15. The water-soluble fullerene according to any of claims 1 to 14 wherein the water-soluble fullerene or the aggregate thereof is in a form of an aqueous solution.

16. A process for producing a water-soluble fullerene **characterized by** reacting a water-soluble polymer with a functional group of the fullerene having the functional group in the molecule.

17. The process for producing a water-soluble fullerene according to claim 16 wherein the water-soluble polymer is any water-soluble polymer of claims 6 to 12.

18. The process for producing a water-soluble fullerene according to claim 16 or 17 wherein the functional group of a fullerene is one carboxyl group.

19. An active oxygen generator which contains a water-soluble fullerene in any of claims 1 to 15 or a water-soluble fullerene produced by a process for producing in any of claims 16 to 18.

20. The active oxygen generator according to claim 19 to be used for photodynamic therapy or sonodynamic therapy.

21. The active oxygen generator according to claim 19 for inhibiting cell proliferation.

22. The active oxygen generator according to claim 21 wherein the cell is a cancer cell.

23. The active oxygen generator according to any of claims 19 to 22 for use in treating cancer.
